# FASCICULE DE BREVET EUROPEEN

(11) **EP 3 893 633 B1**
(45) Date de publication et mention de la délivrance du brevet: **07.12.2022**
(21) Numéro de dépôt: 19842799.9
(22) Date de dépôt: 09.12.2019
(51) Int. Cl.: A01K 67/033, B65G 1/04

(54) **ATELIER D'ÉLEVAGE D'INSECTES COMPORTANT UN VÉHICULE À GUIDAGE AUTOMATIQUE INSTRUMENTÉ**
ANLAGE ZUR ZUCHT VON INSEKTEN MIT EINEM AUTOMATISIERTEN, MIT EINEM SENSOR AUSGERÜSTETEN GEFÜHRTEN FAHRZEUG
FACILITY FOR FARMING INSECTS, COMPRISING AN AUTOMATED SENSOR-EQUIPPED GUIDED VEHICLE

(30) Priorité: 12.12.2018 FR 1872760
(43) Date de publication de la demande: 20.10.2021
(73) Titulaire: Ynsect, 91058 Évry-Courcouronnes Cedex (FR)
(72) Inventeur: DU JONCHAY, Thibault, 60710 Chevrières (FR); ESCAROZ CETINA, Arturo, 5611 BN Eindhoven (NL); COMPARAT, Solene, 95130 Franconville (FR); BERRO, Fabrice, 75001 Paris (FR)
(74) Mandataire: Santarelli
(86) Numéro de dépôt international: PCT/FR2019/052984
(87) Numéro de publication internationale: WO 2020/120889

(56) Documents cités:
- WO-A1-2017/141155
- FR-A1- 3 034 622

## Description

La présente invention concerne le domaine des ateliers d'élevage d'insectes.

Elle vise en particulier les ateliers d'élevage d'insectes présentant un haut degré d'automatisation. Elle vise notamment les ateliers de culture dans lesquels des insectes sont élevés dans des contenants adaptés.

Dans le cadre d'un atelier d'élevage d'insectes, qui servira d'exemple principal pour illustrer la présente invention, les insectes visés par l'invention sont par exemple les coléoptères, les diptères, les lépidoptères, les isoptères, les orthoptères, les hyménoptères, les blattoptères, les hémiptères, les hétéroptères, les éphéméroptères et les mécoptères, de préférence, les coléoptères, les diptères, les orthoptères, les lépidoptères.

Le terme « insecte » est employé pour désigner tout stade d'évolution de l'œuf ou oothèque à l'insecte adulte, et l'invention vise plus particulièrement l'élevage des insectes du stade larvaire à l'insecte adulte.

L'élevage d'insectes connaît un certain essor. La production d'insectes présente de nombreux intérêts, que ce soit pour l'agro-industrie, certaines espèces d'insectes comestibles étant riches en protéines, ou dans d'autres domaines industriels. Typiquement, l'exosquelette des insectes est constitué en grande partie de chitine, dont un dérivé connu est le chitosan. Les applications de la chitine et/ou du chitosan sont nombreuses : cosmétique (composition cosmétique), médicale et pharmaceutique (composition pharmaceutique, traitement des brûlures, biomatériaux, pansements cornéens, fils chirurgicaux), diététique et alimentaire, technique (agent filtrant, texturant, floculant ou adsorbant notamment pour la filtration et dépollution de l'eau), etc. En effet, la chitine et /ou le chitosan sont des matériaux biocompatibles, biodégradables et non toxiques.

Le document FR3034622 présente un atelier adapté à l'élevage d'insectes à une échelle industrielle. L'élevage y met en oeuvre des contenants d'élevage (typiquement des bacs) qui sont empilés, en une ou plusieurs colonnes, pour former des unités élémentaires d'élevage. Les unités élémentaires d'élevage sont stockées, et, lorsqu'une opération d'élevage doit être réalisée, les contenants sont amenés, par des systèmes automatisés tels que des automates, vers un poste adapté à la réalisation de l'opération, groupés en unités élémentaires d'élevage ou dégroupés unitairement.

Les insectes vivent ainsi dans une zone dans laquelle ils croissent et se développent entre les opérations d'élevage. Il est donc important dans cette zone et plus généralement dans l'ensemble de l'atelier d'y faire régner des conditions environnementales favorables à leur santé, leur bien-être, et leur croissance rapide.

Par conditions environnementales, il est notamment fait référence aux paramètres environnementaux que sont la température de l'air, à l'hygrométrie, et au taux de dioxyde de carbone (CO₂) présent dans l'air.

Les conditions environnementales permettent ainsi de juger d'un état de l'atelier, mais d'autres paramètres caractéristiques de cet état de l'atelier doivent être contrôlés pour assurer de bonnes conditions d'élevage. Parmi ces paramètres caractéristiques de l'état de l'atelier on peut notamment citer l'absence (ou au contraire la présence) d'espèces parasites dans l'atelier.

Le document CN107372375 indique de manière générale l'importance de contrôler la température, l'humidité et le taux de CO₂ dans un élevage de vers à soie. Ce document décrit un local d'élevage comportant un capteur de température, d'humidité dans l'air, et de CO₂.Cependant, le seul contrôle de ces paramètres dans un élevage d'insectes à une échelle industrielle se révèle bien insuffisant à de nombreux égards. Le contrôle global proposé ne permet pas de détecter une dérive ou une anomalie locale d'un paramètre environnemental. Un tel événement local est néanmoins susceptible d'affecter une partie de l'élevage, et peut également être le signe d'un événement pouvant affecter l'ensemble de l'élevage. En outre, dans le cadre d'un élevage à très grande échelle, il serait complexe et coûteux de prévoir de couvrir l'ensemble de l'atelier d'élevage par des capteurs.

La présente invention vise ainsi à proposer un atelier d'élevage d'insectes, dans lequel les problématiques évoquées ci-dessous sont résolues en tout ou partie.

En particulier, l'invention porte sur un atelier d'élevage d'insectes, ledit atelier comportant une première zone dans laquelle des insectes sont stockés au cours de leur croissance dans des contenants et une deuxième zone comportant au moins un poste configuré pour la réalisation d'une opération sur les insectes d'un contenant d'élevage ou sur ledit contenant, l'atelier comportant des véhicules à guidage automatique configurés pour le déplacement des contenants d'élevage dans l'atelier, à savoir dans la première zone, et/ou dans la deuxième zone, et entre la première zone et la deuxième zone. Les véhicules à guidage automatique comportent au moins un véhicule à guidage automatique instrumenté, ledit véhicule à guidage automatique instrumenté étant équipé d'au moins un capteur adapté à réaliser une mesure d'un paramètre caractéristique d'un état de l'atelier.

L'invention propose ainsi de tirer profit de l'emploi dans l'atelier de véhicules à guidage automatiques pour le transport des contenants de culture ou d'élevage pour réaliser un contrôle d'un état de l'atelier par la mesure d'au moins un paramètre caractéristique. La solution ainsi proposée a de multiples avantages. Un contrôle aléatoire peut être réalisé au gré des déplacements du véhicule à guidage automatique dans ses missions de transport des contenants. Un contrôle spécifique par exemple dans un endroit souhaité de l'atelier, peut être réalisé au besoin et de manière simple par une programmation adaptée du déplacement d'un véhicule à guidage automatique instrumenté.

Le véhicule à guidage automatique instrumenté peut comporter, en tant que capteur adapté à mesurer un paramètre caractéristique d'un état de l'atelier, un capteur d'humidité, un capteur de température, ou un capteur de taux de dioxyde de carbone. En outre ou alternativement, le véhicule à guidage automatique instrumenté comporte, en tant que capteur adapté à mesurer un paramètre caractéristique d'un état de l'atelier, un capteur de mouvement, une caméra optique et/ou thermique, ou un capteur de résistance au mouvement.

L'état de l'atelier est ainsi défini par exemple par un ou plusieurs paramètres environnementaux régnant dans l'atelier à l'endroit ou une mesure est réalisée, tels que la température, l'humidité de l'air, ou le taux de CO2. Mais d'autres paramètres peuvent être pris en compte. Notamment, la présence d'espèces parasites (notamment d'insectes parasites) dans l'atelier peut être détectée grâce à des paramètres directs, c'est-à-dire leur détection et leur reconnaissance optique, ou par des marqueurs indirects de leur présence.

Le capteur adapté à mesurer un paramètre caractéristique d'un état de l'atelier peut être fixe vis-à-vis d'un corps du véhicule à guidage automatique instrumenté ou, alternativement, le véhicule à guidage automatique instrumenté peut être configuré de sorte que le capteur adapté à mesurer un paramètre caractéristique d'un état de l'atelier peut être orienté ou déplacé vis-à-vis du corps du véhicule à guidage automatique instrumenté.

Le véhicule à guidage automatique instrumenté peut comporter un dispositif de transmission sans fil configuré pour émettre un signal correspondant à la mesure du paramètre caractéristique.

Le véhicule à guidage automatique instrumenté peut comporter une mémoire informatique dans laquelle le paramètre caractéristique est enregistré et un port de communication permettant le déchargement de ladite mémoire par voie filaire ou sans fil.

Un dispositif de transmission sans fil permet la détection en temps réel d'une anomalie dans l'état de l'atelier. Des contrôles supplémentaires ou des mesures correctives peuvent alors être réalisés. Un système permettant le stockage des mesures dans une mémoire permet de différer leur transmission ou de garantir qu'une copie en reste accessible en cas d'échec de la réception. De manière générale, pour des ateliers de grandes dimensions ou pour des raisons économiques ou techniques, il peut être préférable de prévoir une transmission seulement périodique des signaux correspondant aux mesures réalisées.

L'atelier peut comporter un système central de contrôle de l'état de l'atelier adapté à recevoir du véhicule à guidage automatique instrumenté (AGVI) un signal représentatif de la mesure du paramètre caractéristique et à déterminer, en fonction dudit signal représentatif corrélé à une information de position du véhicule à guidage automatique instrumenté lors de ladite mesure, la présence d'une anomalie locale dans l'état de l'atelier.

Ainsi, le contrôle de l'état de l'atelier réalisé de manière conventionnelle par des capteurs fixes peut être complété par les mesures réalisées par le véhicule à guidage automatique instrumenté. Par complété, il s'agit de produire des mesures équivalentes en vue d'une comparaison (par exemple pour confirmer une anomalie détectée par un capteur fixe), de produire des mesures en cas de défaillance d'un capteur fixe, de produire des mesures plus précises (par exemple plus localisées) ou de produire des mesures dans les zones de l'atelier qui ne sont pas couvertes par les capteurs fixes.

L'atelier peut comporter un système central de contrôle de l'état de l'atelier adapté à recevoir du véhicule à guidage automatique instrumenté un signal représentatif de la mesure du paramètre caractéristique et à déterminer, en fonction dudit signal représentatif corrélé à une information de position du véhicule à guidage automatique instrumenté lors de ladite mesure, la présence d'une anomalie locale dans l'état de l'atelier.

Le système central de contrôle de l'état de l'atelier peut en outre être adapté à recevoir des capteurs fixes des signaux représentatifs de mesures du paramètre caractéristique et à déterminer la présence d'une anomalie locale dans l'état de l'atelier en fonction des signaux représentatifs, chaque signal représentatif étant corrélé à une information d'identité du capteur fixe qui l'émet.

L'atelier peut comporter un système central de guidage, ledit système central de guidage étant adapté à transmettre des informations de guidage auxdits véhicules à guidage automatique et notamment audit véhicule à guidage automatique instrumenté.

Le système central de guidage peut être configuré pour transmettre au véhicule à guidage automatique instrumenté des informations de guidage en réponse à une anomalie locale déterminée sur le fondement d'un signal émis par l'un des capteurs fixes.

Le système central de guidage peut être configuré pour transmettre périodiquement au véhicule à guidage automatique instrumenté des informations de guidage afin de lui faire inspecter une partie de l'atelier qui n'est pas couverte par un capteur fixe de l'atelier pour y mesurer le paramètre caractéristique.

Le système central de guidage peut être configuré pour transmettre périodiquement au véhicule à guidage automatique instrumenté, des informations de guidage afin de lui faire inspecter une partie de l'atelier dans laquelle les véhicules à guidage automatique ne transitent pas lors du transport des contenants.

Le contrôle et la gestion de l'état de l'atelier peuvent ainsi être envisagés de manière globale. Notamment, le système central de contrôle de l'état de l'atelier peut être interfacé avec le système contrôlant les déplacements des véhicules à guidage automatique. Les déplacements du ou des véhicules à guidage automatique instrumentés peuvent ainsi être gérés pour assurer de manière optimales tant leurs missions de transport que des missions de contrôle des paramètres à surveiller.

L'invention porte également sur un procédé de contrôle d'un état d'un atelier d'élevage d'insectes comportant une première zone dans laquelle des insectes sont stockés au cours de leur croissance dans des contenants et une deuxième zone comportant au moins un poste configuré pour la réalisation d'une opération sur les insectes d'un contenant ou sur ledit contenant, l'atelier comportant des véhicules à guidage automatique configurés pour le déplacement des contenants dans l'atelier, à savoir dans la première zone et/ou dans la deuxième zone , et entre la première zone et la deuxième zone, ledit procédé étant caractérisé par la mesure d'un paramètre caractéristique de l'état de l'atelier par un véhicule à guidage automatique instrumenté équipé d'au moins un capteur adapté à réaliser la mesure dudit paramètre caractéristique de l'état de l'atelier.

D'autres particularités et avantages de l'invention apparaîtront encore dans la description ci-après.
Aux dessins annexés, donnés à titre d'exemples non limitatifs :
la figure 1 représente selon une vue tridimensionnelle schématique un exemple d'atelier d'élevage d'insectes conforme à un exemple de mode de réalisation de l'invention ;
la figure 2 représente, selon une vue schématique en trois dimensions, un ensemble de contenants d'élevage pouvant être employé dans un élevage d'insectes ;
la figure 3 représente un premier exemple de véhicule à guidage automatique pouvant être mis en oeuvre dans l'invention ;
la figure 4 représente un deuxième exemple de véhicule à guidage automatique pouvant être mis en oeuvre dans l'invention ;
la figure 5 représente selon un schéma de principe un exemple des flux de données pouvant être établis dans un atelier conforme à un mode de réalisation de l'invention.

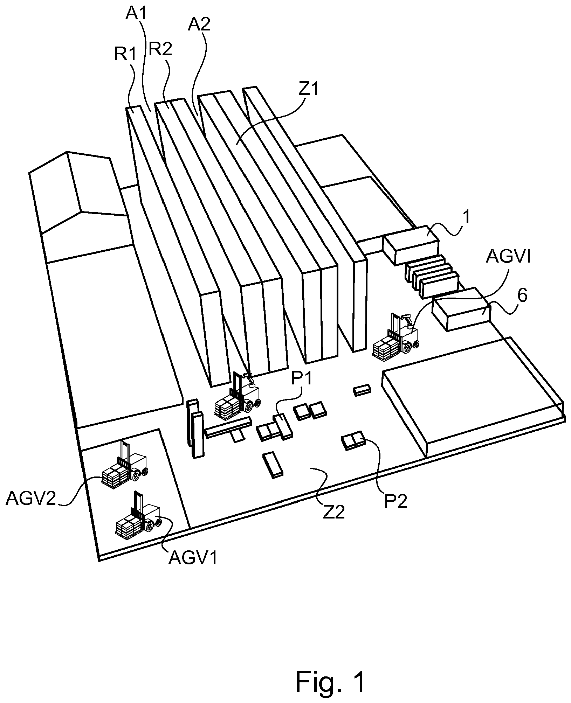

La figure 1 représente un atelier d'élevage d'insectes, ici représenté sous la forme d'une vue schématique en trois dimensions.

L'élevage d'insectes peut notamment être envisagé comme un ensemble organisé permettant la ponte d'œufs par des insectes adultes pour la production de larves, certaines larves étant élevées jusqu'au stade adulte pour la ponte de nouveaux œufs, les adultes étant renouvelés régulièrement (par exemple suite à leur mort) par des adultes jeunes assurant de nouvelles pontes et ainsi de suite. Le produit final de la production peut être des œufs, et/ou des larves, et/ou des nymphes, et/ou des insectes adultes.

Tel que représenté à la figure 1, un atelier d'élevage d'insectes conforme à l'invention, ici représenté de manière schématique en trois dimensions, comporte au moins deux zones, à savoir une première zone Z1 organisée pour le stockage des insectes pendant leur croissance. Dans cette première zone Z1, les insectes grandissent dans des conditions environnementales (définies par des paramètres environnementaux dont la température, l'hygrométrie...) contrôlées et optimisées.

L'atelier comporte également une seconde zone Z2, organisée pour la réalisation d'une ou plusieurs opérations d'élevage. Les opérations d'élevage correspondent à des opérations devant être menées pour le maintien en vie, la bonne croissance, et/ou l'optimisation des conditions d'élevage des insectes.

La récolte des insectes constitue une opération pouvant être menées dans la seconde zone Z2.

La seconde zone Z2 comporte en particulier, dans l'exemple représenté à la figure 1, un ou plusieurs postes de travail P1, P2, spécialisés dans la réalisation d'une ou plusieurs opérations d'élevage.

Les insectes (œufs, larves, nymphes, ou adultes) sont élevés dans des contenants, pouvant être groupés en ensembles appelés unités élémentaires d'élevage. Lors de phases de croissance, les contenants sont stockés dans la zone de stockage Z1, par exemple dans des rayonnages R1, R2 à palettes.

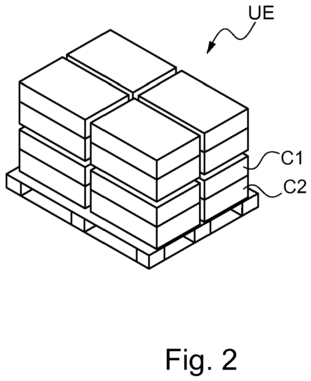

Un exemple d'unité élémentaire d'élevage est représenté à la figure 2 selon une représentation de principe en trois dimensions. Afin d'en faciliter la manutention, chaque unité élémentaire d'élevage peut être portée par une palette, comme représenté à la figure 2.

En particulier, les contenants C1, C2 d'élevage peuvent être des caisses ou bacs empilables. Par bacs ou caisses empilables, on désigne en particulier des bacs ou caisses qui se superposent les unes au-dessus des autres, de manière légèrement encastrée, ce qui procure une certaine stabilité à la colonne de caisses ainsi formée.

Tel que représenté à la figure 2, les contenants C1, C2 sont palettisés, c'est-à-dire groupés en unités élémentaires UE sur une palette de manutention. La palette peut notamment, mais pas exclusivement, être une palette de taille classique, c'est-à-dire typiquement une palette de type « palette Europe », ou une demi-palette de ce type.

A titre d'exemple, une unité élémentaire UE d'élevage peut typiquement regrouper de huit à cent contenants C1, C2, et comporter une, deux, trois, quatre piles de contenants, voire plus. La hauteur d'une unité élémentaire d'élevage complète peut par exemple être comprise entre 160 et 230 cm, et typiquement de l'ordre de 200cm.

Dans l'exemple d'organisation représenté à la figure 1, les rayonnages à palettes sont séparés par des allées A1, A2 parallèles permettant la circulation entre les rayonnages R1, R2. Les contenants d'élevage, par exemple sous forme de caisses unitaires ou rassemblés en unités élémentaires, doivent être transportés dans l'atelier à plusieurs reprises pendant l'élevage. En particulier, les contenants doivent être récupérés dans la première zone Z1 pour être transportés dans la seconde zone Z2 ou des opérations sont menées. De même, les contenants issus de la seconde zone Z2 doivent généralement être transportés dans la première zone Z1 pour y être stockés, par exemple dans les rayonnages à palettes ou dans des structures analogues.

Par ailleurs, un transport des contenants, sous forme palettisées ou non, peut être nécessaire à l'intérieur même de la première zone Z1, ou entre différents postes de la deuxième zone Z2.

Pour cela, l'atelier comporte des véhicules à guidage automatique AGV1, AGV2. Les véhicules à guidage automatique ou véhicules autonomes sont souvent désignés par l'acronyme anglophone AGV pour « automatic guided vehicles ». Il s'agit, dans un contexte industriel, d'automates pouvant se déplacer conformément à des informations de guidages pouvant être fournies auxdits véhicules à guidage automatique AGV1, AGV2 par différents moyens. Le véhicule peut mettre en oeuvre diverses technologies d'automatisation pour son guidage, qui peut être réalisé par filoguidage, optoguidage, et/par des technologies plus souples fondées sur la géolocalisation et le géoguidage du véhicule dans l'atelier. Le cas échéant une ou plusieurs caméras ou systèmes à ultrasons permettent au véhicule de percevoir son environnement spatial et d'adapter ses déplacements en fonction de ce dernier.

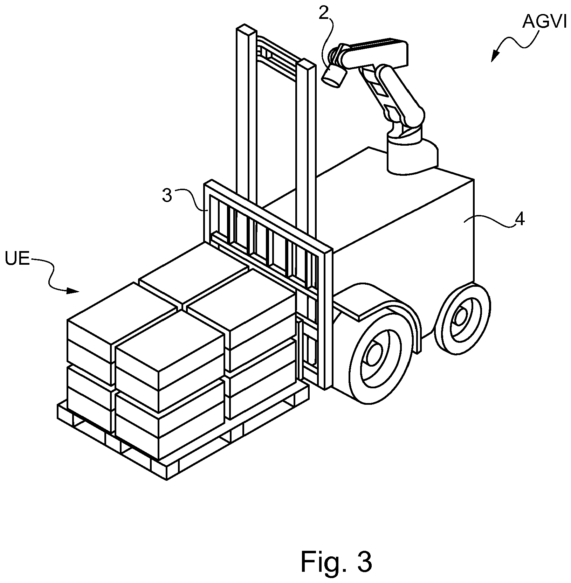

Les véhicules à guidage automatique mis en oeuvre peuvent être de divers types. Dans le cadre d'un atelier mettant en oeuvre des unités élémentaires sous la forme d'ensembles de contenants palettisés, les véhicules peuvent avantageusement avoir la forme d'un transpalette automatisé, tel que représenté et décrit plus avant ci-après en référence à la figure 3.

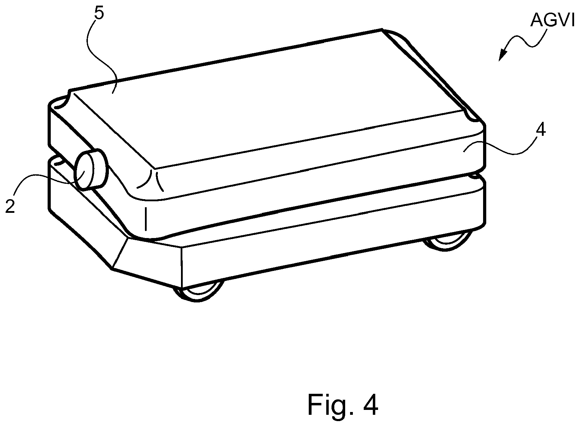

Des véhicules automatisés plus simples peuvent être employés, par exemple pour le transport de contenants unitaires ou sous forme d'une simple pile de caisses. De tels véhicules à guidage automatique sont illustrés et décrits ci-après en référence à la figure 4.

Les véhicules à guidage automatique mis en oeuvre dans l'invention ont en premier lieu le rôle d'assurer les flux de contenants dans l'atelier d'élevage d'insectes ; c'est-à-dire de transporter les contenants selon les besoins. A cette fin, un dispositif central de guidage 1 peut être prévu. Le dispositif central de guidage 1 comporte les moyens informatiques nécessaires à planifier les déplacements de chaque véhicule à guidage automatique, et à transmettre audit véhicule les informations nécessaires son guidage. Ces informations peuvent consister en un trajet complet que doit réaliser un véhicule à guidage automatique donné, ou, si des technologies tels que le filoguidage ou l'optoguidage sont utilisées, ces informations peuvent consister en un ordre de suivre une piste donnée. Les informations contiennent également l'indication des contenants à récupérer, ainsi que leur destination.

Ainsi, le système central de guidage 1 permet d'affecter individuellement des missions de transport aux véhicules à guidage automatique AGV1, AGV2.

La transmission des informations de guidage est avantageusement réalisée par des moyens de communication sans fil. Le véhicule à guidage automatique AGV1, AGV2 peut également transmettre des informations en retour au dispositif central de guidage 1. Notamment, dans le cas d'un véhicule géolocalisé dans l'atelier, le dispositif central de guidage peut recevoir des informations de capteurs de localisation présents dans l'atelier mais aussi du véhicule à guidage automatique lui-même. Si le véhicule à guidage automatique est doté d'une caméra ou d'un dispositif à ultrason, ou laser, pour son guidage, les données issues de ce dispositif peuvent être transmis au dispositif central de guidage qui pourra transmettre en réponse de nouvelles informations de guidage. Ainsi, une grande partie de la composante logicielle, voire de l'intelligence, nécessaire au guidage des véhicules à guidage automatique peut être centralisée au niveau du dispositif central de guidage.

Un atelier conforme à la présente invention comporte parmi les véhicules à guidage automatique au moins un véhicule à guidage automatique instrumenté AGVI.

Le véhicule à guidage automatique est dit instrumenté en ce qu'il comporte un capteur adapté à réaliser une mesure d'un paramètre caractéristique d'un état de l'atelier.

Par état de l'atelier, il est fait référence certains critères et conditions propices ou au contraire inappropriés pour l'élevage des insectes élevés dans l'atelier.

Par paramètre caractéristique d'un état de l'atelier, il est ainsi fait référence notamment aux paramètres environnementaux pouvant être classiquement surveillés, à savoir la température, l'humidité de l'air, et la teneur en dioxyde de carbone. Ces paramètres traduisent directement certaines conditions propices ou inappropriées à la vie des insectes de l'élevage, ou à leur croissance. Ils peuvent également caractériser des conditions favorables à l'apparition de moisissure, de champignons, de maladies fongiques des insectes élevés.

Mais d'autres critères peuvent être contrôlés sur le fondement de ces paramètres environnementaux ou d'autres paramètres caractéristiques. Notamment, l'absence de certains polluants dans l'air de l'atelier (monoxyde de carbone, oxyde d'azote, ammoniac, méthane, produits soufrés volatils tels que le sulfure d'hydrogène) peut être contrôlée à l'aide d'un capteur adapté. De même, la présence de certains spores dans l'air peut être détectée à l'aide d'un capteur adapté, et plus généralement une détection de la charge microbienne peut être prévue.

De même, la luminosité peut être pour certains élevages un paramètre important.

En outre, parmi les caractéristiques d'un état de l'atelier, il est important de contrôler la présence, ou l'absence, d'espèces parasites dans l'atelier. Notamment, certains diptères ou certains arachnides peuvent se développer dans l'élevage. Ces espèces parasites, non souhaitées dans l'atelier, peuvent être vecteurs de maladies pour les insectes de l'élevage , ou en devenir concurrentes au moins dans certaines zones de l'élevage.

De telles espèces peuvent être détectées directement, par exemple à l'aide d'une caméra. Une espèce peut être détectée par reconnaissance d'image. Certaines espèces peuvent être détectées même en l'absence de reconnaissance d'image, par exemple par la détection de la trajectoire d'un objet dans les images issues de la caméra. Il est notable à cet égard, qu'une même caméra peut être employée pour le guidage du véhicule à guidage automatique et pour la détection d'un paramètre caractéristique de l'état de l'atelier, par exemple la présence d'une espèce parasite. Mais dans ce cas le véhicule à guidage automatisé instrumenté se distingue des véhicules à guidage automatique classique en ce que son capteur est adapté, dans le traitement des données qui en sont issues, pour mesurer la présence ou l'absence d'une espèce parasite. Une caméra dédiée peut aussi être employée, que le véhicule à guidage automatique soit équipé ou non d'une caméra pour son guidage.

Alternativement ou en complément, un capteur de mouvement équipant le véhicule à guidage automatique instrumenté peut être employé pour détecter la présence d'une espèce parasite.

Par ailleurs, de telles espèces peuvent être détectées par des marqueurs indirects de leur présence. Un de ces marqueurs peut être la présence de soies ou toiles, par exemple détectées mécaniquement par la résistance au mouvement qu'elle provoque. Le véhicule à guidage automatique instrumenté peut comporter à cette fin un capteur de force permettant de détecter une résistance au mouvement (par exemple à l'avancement du véhicule, ou une résistance lors du mouvement d'un bras mobile que comporte le véhicule à guidage automatique instrumenté). Une détection optique des soies ou toiles peut aussi être mise en oeuvre.

Un autre marqueur indirect peut être la chaleur provoquée par le mouvement analogue à un mouvement brownien de ces espèces parasites. Il en résulte un point, ou une petite zone, dont la température est supérieure à celle de son environnement immédiat. Un capteur de température permettant de mesurer localement une température permet cette caractérisation, de même qu'une caméra thermique.

La figure 3 représente un véhicule à guidage automatique pouvant être employé dans un atelier conforme à un mode de réalisation de l'invention. Le véhicule à guidage automatique représenté est en particulier un véhicule à guidage automatique instrumenté AGVI en ce qu'il est équipé d'un capteur 2.

Le véhicule à guidage automatique représenté est du type transpalette à guidage automatique. Ce véhicule est particulièrement adapté au transport de palettes, en ce qu'il comporte une fourche configurée pour permettre de soulever et maintenir une palette classique. Ce véhicule est également configuré, grâce à un système de levage 3, pour aller récupérer des palettes en partie haute des rayonnages R1, R2 à palettes.

A la figure 3, le véhicule à guidage automatique est représenté portant une unité élémentaire UE palettisée.

Le capteur 2 peut être de tout type mentionné ci-avant. Il peut être par exemple une caméra thermique. Le capteur 2 est mobile vis-à-vis du corps 4 ou châssis du véhicule à guidage automatique. A cette fin, dans l'exemple représenté le capteur est monté sur un bras robotisé mobile sur plusieurs axes. Ce type de montage offre une mobilité en rotation et en translation au capteur 2. Le capteur 2 peut ainsi être orienté vers un point d'intérêt particulier, ou être éloigné ou rapproché d'un tel point d'intérêt.

D'autres systèmes permettant de rendre le capteur mobile peuvent être envisagés dans le cadre de l'invention : capteur monté sur un simple pivot, bras télescopique pivotant ou non, etc.

Plusieurs capteurs peuvent équiper un même véhicule à guidage automatique instrumenté AGVI. Les capteurs peuvent dans ce cas être montés d'un seul et même système assurant leur mobilité, ou sur des systèmes différents. Certains capteurs peuvent être mobiles, et d'autres fixes.

A la figure 4, le véhicule à guidage automatique représenté comporte un capteur 2 fixe. Fixe s'entend vis-à-vis du corps 4 du véhicule. Le véhicule à guidage automatique représenté à la figure 4 est d'un type à plateau de charge 5. Le plateau de charge 5 permet d'y poser une charge à transporter. Cette charge peut être un contenant (d'élevage ou de culture), une pile de contenants, une unité élémentaire le cas échéant palettisée, bien que dans le cadre d'un atelier mettant en oeuvre des ensembles palettisés un type de véhicule à guidage automatique plus avantageux peut être choisi.

Bien évidemment, le véhicule à guidage automatique du type représenté à la figure 4 peut être équipé d'un ou plusieurs capteurs mobiles, par exemples rendus mobiles à l'aide d'un des systèmes évoqués ci-avant, en alternative ou en complément à un ou plusieurs capteurs fixes.

Par exemple, dans le mode de réalisation de la figure 4, le capteur 2 peut être une caméra, ayant une fonction de correction du guidage du véhicule à guidage automatique (par exemple pour l'évitement d'un obstacle) et étant par ailleurs adapté à mesurer un paramètre caractéristique d'un état de l'atelier, par exemple à détecter la présence d'insectes parasites volants ou rampants.

Les capteurs équipant le ou les véhicules à guidage automatique instrumentés que comporte l'atelier (tous les véhicules à guidage automatique de l'atelier peuvent être instrumentés, ou seulement certains de ces véhicules) permettent de réaliser des mesures au gré des déplacements des véhicules à guidage automatique instrumentés dans le cadre du transport des contenants, et/ou le cas échéant lors de trajets réalisés dans l'atelier avec pour objectif principal de réaliser de telles mesures.

L'atelier, par exemple l'atelier représenté à la figure 1, peut comporter des capteurs fixes pour la surveillance de l'état de l'atelier. Généralement, l'atelier peut être équipé de capteur des conditions environnementales que sont la température, le taux d'humidité, et/ou le taux de dioxyde de carbone dans l'air. Les mesures réalisées par les véhicules à guidage automatique permettent de compléter dans ce cas les mesures réalisées par les capteurs fixes de l'atelier. Par « compléter », il peut s'agir de mesurer à l'aide d'un véhicule à guidage automatique instrumenté un même paramètre que celui qui est mesuré par un capteur fixe de l'atelier, ou un paramètre intimement lié à ce dernier, et de vérifier la concordance des mesures respectivement réalisées par le capteur fixe de l'atelier et le capteur du véhicule à guidage automatique instrumenté. Il peut également s'agir de compléter spatialement les mesures des capteurs fixes de l'atelier, par exemple en mesurant le paramètre caractéristique dans une partie de l'atelier qui n'est pas couverte par les capteurs fixes. Il peut enfin s'agir de compléter qualitativement les mesures des capteurs fixes de l'atelier. En d'autres termes, il peut également s'agir de produire des mesures d'un paramètre qui n'est pas mesuré par les capteurs fixes. Par exemple les capteurs fixes de l'atelier peuvent mesurer la température, le taux d'humidité, et le taux de CO₂, tandis que le véhicule à guidage automatique instrumenté permet de détecter par exemple la présence d'une espèce parasite, ou de détecter le développement d'un champignon dans l'atelier (que ce soit dans les contenants ou non, par exemple sur les murs de l'atelier).

Pour assurer un contrôle efficace d'un ou plusieurs paramètres caractéristiques de l'état de l'atelier, l'atelier comporte avantageusement un système central de contrôle de l'état de l'atelier 6. Le système central de contrôle comporte un ensemble de systèmes électroniques ou informatiques adapté à récupérer, par exemple sous la forme de signaux représentatifs, les mesures réalisées par les véhicules à guidage automatique, et par les capteurs fixes de l'atelier lorsque ce dernier en comporte. Les mesures sont traitées par le système central de contrôle afin par exemple de détecter une anomalie locale, pouvant traduire un événement particulier ou redouté. L'anomalie peut consister en la mesure d'une valeur du paramètre mesuré en dehors d'une plage de valeur acceptable, ou en la détection directe d'un événement (mouvement, point chaud, présence d'une soie ou toile traduisant la présence d'une espèce parasite dans l'atelier).

Le système central de contrôle de l'état de l'atelier 6 est avantageusement interfacé avec le système central de guidage. L'interfaçage entre le système central de contrôle de l'état de l'atelier et le système central de guidage consiste à permettre un échange d'informations entre ces systèmes, de sorte par exemple que des informations sur la position et le trajet de chaque véhicule à guidage automatique, instrumenté ou non, puisse être pris en considération par le système central de contrôle de l'état de l'atelier pour piloter des mesures en vue du contrôle de l'état de l'atelier En effet, l'un des intérêts de mettre en oeuvre des véhicules à guidage automatique instrumentés est de réaliser une mesure au besoin. Notamment, un véhicule à guidage automatique instrumenté peut être envoyé à un endroit donné de l'atelier où une mesure issue d'un capteur fixe de l'atelier est considérée anormale. En outre, un véhicule à guidage automatique instrumenté peut être envoyé périodiquement pour réaliser la mesure du (ou des) paramètre caractéristique dans une partie de l'atelier qui n'est pas couverte par un capteur fixe. En outre, un véhicule à guidage automatique instrumenté peut être envoyé périodiquement pour réaliser la mesure du (ou des) paramètre caractéristique dans une partie de l'atelier qui n'est jamais ou que rarement sur le parcours des véhicules à guidage automatique de l'atelier dans le cadre de leurs missions de transport de contenant. Enfin, lorsque le système central de contrôle de l'état de l'atelier constate, grâce à des informations issues du système central de guidage, qu'aucun véhicule à guidage instrumenté n'est allé dans une partie donnée de l'atelier au gré de ses missions de transport de contenants d'élevage, il peut décider d'en envoyer un, dans le cadre d'une mission de transport de contenants ou le cas échéant spécifiquement pour réaliser un contrôle.

La transmission des données depuis chaque véhicule à guidage automatique instrumenté AGVI vers le système central de contrôle de l'état de l'atelier 6 (et le cas échéant depuis le système central de contrôle vers un véhicule à guidage automatique instrumenté) est réalisé préférentiellement par des moyens de communication sans fil. Les moyens de communication sans fil envisagés incluent notamment des systèmes de communication mettant en oeuvre au moins l'un des protocoles suivants : NFC, RFID, Bluetooth, notamment Bluetooth Low Energy, Lifi, et Zigbee (marques déposées). D'autres protocoles peuvent évidemment être employés.

Selon les besoins et le protocole employé, les communications entre véhicule à guidage automatique instrumenté AGVI et système central de contrôle 6 peuvent être réalisée de manières continues ou seulement périodiquement.

Par « de manière continue », il est notamment envisagé que les mesures soient instantanément transmises à destination du système central de contrôle de l'état de l'atelier 6, par exemple au rythme de la fréquence d'acquisition du ou des capteurs équipant le véhicule.

« Périodiquement » correspond à une transmission discontinue, par exemple à intervalle temporel donné, ou lorsque le véhicule à guidage automatique passe en un point déterminé, à portée de transmission du système central de contrôle de l'élevage ou d'un relais de réception, ou encore lorsqu'il est en station fixe par exemple lorsque le véhicule à guidage automatique est électrique à batteries et est en cours de charge.

Il peut également être prévu, pour une transmission périodique, de transmettre les données au système central de contrôle de l'état de l'atelier par voie filaire, via un port physique du véhicule à guidage automatique instrumenté. Une communication filaire peut également permettre la programmation du véhicule à guidage automatique instrumenté, pour ce qui concerne son guidage ou les mesures qu'il doit effectuer, en particulier lorsque le système central de guidage et le système central de contrôle de l'état de l'atelier sont interfacés.

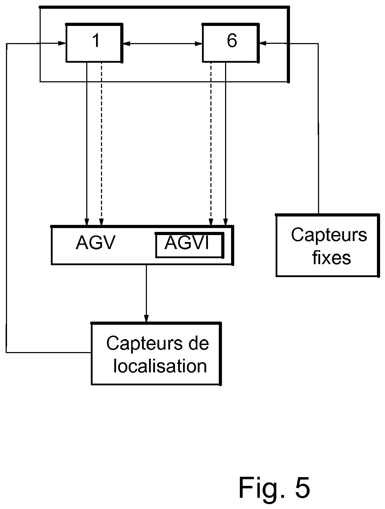

La figure 5 illustre un exemple des flux de données pouvant être mis en oeuvre dans un exemple d'atelier. Pour cet exemple, on considère un atelier d'élevage d'insectes comportant des véhicules à guidage automatique AGV dont certains sont instrumentés. Les véhicules à guidage automatique peuvent être localisés par des capteurs de localisation présents dans l'atelier. L'atelier comporte un système central de guidage 1 et un système central de contrôle de l'état de l'atelier 6. Des capteurs fixes sont disposés dans l'atelier.

Le système central de guidage 1 et le système central de contrôle de l'état de l'atelier 6 sont interfacés de sorte que le système central de guidage 1 fournit au système central de contrôle de l'état de l'atelier 6 des données relatives à la position des véhicules à guidage automatique instrumentés dans l'atelier, ainsi qu'aux trajets auxquels ils sont affectés. Le système central de contrôle de l'état de l'atelier 6 peut envoyer au système central de guidage 1 des instructions afin que le système central de guidage affecte un trajet donné, ou un trajet passant dans une partie donnée de l'atelier, à un véhicule à guidage automatique instrumenté. Une telle affectation peut par exemple être commandée lorsqu'un capteur fixe de l'atelier transmet au système central de contrôle d'un état de l'atelier 6 une mesure anormale, ou pour réaliser la mesure d'un paramètre dans une partie de l'atelier dans laquelle une mesure doit être périodiquement réalisée, que cette partie de l'atelier soit ou non couverte par des capteurs fixes, et que cette partie de l'atelier soit ou non une partie dans laquelle circulent des véhicules à guidage automatique dans le cadre d'une mission de transport de contenants.

Ainsi, le système central de guidage 1 transmet individuellement aux véhicules à guidage automatique AGV, et notamment aux véhicules à guidage automatique instrumentés AGVI, les données nécessaires à leurs déplacements dans l'atelier. En retour, les véhicules à guidage automatique, instrumentés ou non, peuvent le cas échéant transmettre des données relatives à leur déplacement au système central de guidage 1. Ces données peuvent être par exemple une information sur leur mouvement, afin de permettre leur localisation ou confirmer leur localisation, ou des informations sur la réalisation certaines tâches en tant qu'étapes dans le trajet affecté à un véhicule à guidage automatique donné. Ces données peuvent aussi être un retour en temps réel d'informations permettant d'adapter le guidage des véhicules, par exemple un retour vidéo.

Pour la localisation dans l'atelier des véhicules à guidage automatique instrumentés, il est prévu dans l'exemple ici représenté que l'atelier comporte un ensemble de capteurs de localisation. Il peut s'agir de capteurs permettant la réception d'un signal de position émis par les véhicules à guidage automatique, par exemple un signal RFID (de l'anglais radio frequency identification), ou de capteurs qui permettent simplement de constater le passage ou la présence d'un véhicule à guidage automatique et d'en informer le système central de guidage, qui peut corréler cette information aux trajets affectés à chaque véhicule à guidage automatique.

Les véhicules à guidage automatique instrumentés AGVI sont, pour ce qui les concerne, adaptés à transmettre les mesures qu'ils réalisent au système central de contrôle de l'état de l'atelier 6. Cette transmission peut être réalisée selon diverses modalités et divers protocoles évoqués ci-avant.

En retour, le système central de contrôle de l'état de l'atelier 6 peut éventuellement transmettre des informations relatives aux mesures à effectuer, soit directement, soit via les informations de guidage issues du système central de guidage 1 interfacé.

La figure 5 est bien évidemment un simple exemple des flux de données pouvant être mis en oeuvre dans un atelier d'élevage d'insectes conforme à un mode de réalisation de l'invention. D'autres configurations sont possibles, l'atelier d'élevage d'insectes pris comme base de cet exemple comportant plusieurs dispositifs optionnels dans le cadre de l'invention.

L'invention ainsi développée permet l'optimisation du contrôle de l'état d'un atelier d'élevage d'insectes, en tirant partie des moyens utilisés pour le transport des contenants dans l'atelier. En particulier, en instrumentant un ou plusieurs véhicules à guidage automatique, une grande souplesse dans les contrôles peut être obtenue, ainsi que, le cas échéant, la couverture de l'ensemble de l'atelier. En outre, l'emploi de systèmes prévus dans l'atelier pour la fonction de transport permet l'obtention d'une fonction de contrôles de l'état de l'atelier a un coût moindre que l'emploi de systèmes entièrement dédiés au contrôle.

## Revendications

1. Atelier d'élevage d'insectes, ledit atelier comportant une première zone (Z1) dans laquelle des insectes sont stockés au cours de leur croissance dans des contenants (C1, C2) et une deuxième zone (Z2) comportant au moins un poste (P1,P2) configuré pour la réalisation d'une opération sur les insectes d'un contenant ou sur ledit contenant ;
l'atelier comportant des véhicules à guidage automatique (AGV) configurés pour le déplacement des contenants dans l'atelier, à savoir dans la première zone (Z1), et/ou dans la deuxième zone (Z2), et entre la première zone (Z1) et la deuxième zone (Z1),
**caractérisé en ce que** lesdits véhicules à guidage automatique (AGV) comportent au moins un véhicule à guidage automatique instrumenté (AGVI), ledit véhicule à guidage automatique instrumenté (AGVI) étant équipé d'au moins un capteur (2) adapté à réaliser une mesure d'un paramètre caractéristique d'un état de l'atelier.

2. Atelier d'élevage d'insectes selon la revendication 1, dans lequel le véhicule à guidage automatique instrumenté (AGVI) comporte, en tant que capteur (2) adapté à mesurer un paramètre caractéristique d'un état de l'atelier, un capteur d'humidité, un capteur de température, ou un capteur de taux de dioxyde de carbone.

3. Atelier d'élevage d'insectes selon la revendication 1 ou la revendication 2, dans lequel le véhicule à guidage automatique instrumenté (AGVI) comporte, en tant que capteur (2) adapté à mesurer un paramètre caractéristique d'un état de l'atelier, un capteur de mouvement, une caméra optique et/ou thermique, ou un capteur de résistance au mouvement.

4. Atelier d'élevage d'insectes selon l'une des revendications précédentes, dans lequel le capteur (2) adapté à mesurer un paramètre caractéristique d'un état de l'atelier est fixe vis-à-vis d'un corps (4) du véhicule à guidage automatique instrumenté (AGVI).

5. Atelier d'élevage d'insectes selon l'une des revendications 1 à 3, dans lequel le capteur (3) adapté à mesurer un paramètre caractéristique d'un état de l'atelier peut être orienté ou déplacé vis-à-vis d'un corps (4) du véhicule à guidage automatique instrumenté (AGVI).

6. Atelier d'élevage d'insectes selon l'une des revendications précédentes, dans lequel le véhicule à guidage automatique instrumenté (AGVI) comporte un dispositif de transmission sans fil configuré pour émettre un signal correspondant à la mesure du paramètre caractéristique.

7. Atelier d'élevage d'insectes selon l'une des revendications précédentes, dans lequel le véhicule à guidage automatique instrumenté (AGVI) comporte une mémoire informatique dans laquelle le paramètre caractéristique est enregistré et un port de communication permettant le déchargement de ladite mémoire par voie filaire ou sans fil.

8. Atelier d'élevage d'insectes selon l'une des revendications précédentes, comportant en outre des capteurs fixes de mesure du paramètre caractéristique d'un état de l'atelier.

9. Atelier d'élevage d'insectes selon l'une des revendications précédentes, comportant un système central de contrôle de l'état de l'atelier (6) adapté à recevoir du véhicule à guidage automatique instrumenté (AGVI) un signal représentatif de la mesure du paramètre caractéristique et à déterminer, en fonction dudit signal représentatif corrélé à une information de position du véhicule à guidage automatique instrumenté lors de ladite mesure, la présence d'une anomalie locale dans l'état de l'atelier.

10. Atelier d'élevage d'insectes selon les revendications 8 et 9, dans lequel le système central de contrôle de l'état de l'atelier (6) est en outre adapté à recevoir des capteurs fixes des signaux représentatifs de mesures du paramètre caractéristique et à déterminer la présence d'une anomalie locale dans l'état de l'atelier en fonction des signaux représentatifs, chaque signal représentatif étant corrélé à une information d'identité du capteur fixe qui l'émet.

11. Atelier d'élevage d'insectes selon la revendication 9 ou la revendication 10, comportant un système central de guidage (1), ledit système central de guidage (1) étant adapté à transmettre des informations de guidage auxdits véhicules à guidage automatique (AGV) et notamment audit véhicule à guidage automatique instrumenté.

12. Atelier d'élevage d'insectes selon les revendications 10 et 11 dans lequel ledit système central de guidage (1) est configuré pour transmettre au véhicule à guidage automatique instrumenté (AGVI) des informations de guidage en réponse à une anomalie locale déterminée sur le fondement d'un signal émis par l'un des capteurs fixes.

13. Atelier d'élevage d'insectes 10 et 11, dans lequel ledit système central de guidage (1) est configuré pour transmettre périodiquement au véhicule à guidage automatique instrumenté (AGVI) des informations de guidage afin de lui faire inspecter une partie de l'atelier qui n'est pas couverte par un capteur fixe de l'atelier pour y mesurer le paramètre caractéristique.

14. Atelier d'élevage d'insectes selon la revendication 11 ou la revendication 12, dans lequel ledit système central de guidage (1) est configuré pour transmettre périodiquement au véhicule à guidage automatique instrumenté (AGVI), des informations de guidage afin de lui faire inspecter une partie de l'atelier dans laquelle les véhicules à guidage automatique ne transitent pas lors du transport des contenants (C1,C2).

15. Procédé de contrôle d'un état d'un atelier d'élevage d'insectes comportant une première zone (Z1) dans laquelle des insectes sont stockés au cours de leur croissance dans des contenants (C1, C2) et une deuxième zone (Z2) comportant au moins un poste (P1,P2) configuré pour la réalisation d'une opération sur les insectes d'un contenant ou sur ledit contenant, l'atelier comportant des véhicules à guidage automatique (AGV) configurés pour le déplacement des contenants dans l'atelier, à savoir dans la première zone (Z1), et/ou dans la deuxième zone (Z2), et entre la première zone (Z1) et la deuxième zone (Z1),
ledit procédé étant **caractérisé par** la mesure d'un paramètre caractéristique de l'état de l'atelier par un véhicule à guidage automatique instrumenté (AGVI) équipé d'au moins un capteur adapté à réaliser la mesure dudit paramètre caractéristique de l'état de l'atelier.

## Patentansprüche

1. Anlage zur Zucht von Insekten, wobei die Anlage einen ersten Bereich (Z1) aufweist, in dem Insekten während ihres Wachstums in Behältern (C1, C2) gelagert sind, und einen zweiten Bereich (Z2), der mindestens einen Posten (P1, P2) aufweist, der für die Durchführung einer Maßnahme für die Insekten eines Behälters oder für den Behälter ausgelegt ist;
wobei die Anlage fahrerlose Transportfahrzeuge (AGV) aufweist, die für die Verlagerung der Behälter in der Anlage ausgelegt sind, nämlich im ersten Bereich (Z1) und/oder im zweiten Bereich (Z2) und zwischen dem ersten Bereich (Z1) und dem zweiten Bereich (Z1),
**dadurch gekennzeichnet, dass** die fahrerlosen Transportfahrzeuge (AGV) mindestens ein instrumentiertes fahrerloses Transportfahrzeug (AGVI) aufweisen, wobei das instrumentierte fahrerlose Transportfahrzeug (AGVI) mit mindestens einem Sensor (2) ausgestattet ist, der zur Durchführung einer Messung eines für einen Zustand der Anlage charakteristischen Parameters geeignet ist.

2. Anlage zur Zucht von Insekten nach Anspruch 1, wobei das instrumentierte fahrerlose Transportfahrzeug (AGVI) als Sensor (2), der zur Messung eines für einen Zustand der Anlage charakteristischen Zustands geeignet ist, einen Feuchtigkeitssensor, einen Temperatursensor oder einen Sensor des Kohlendioxidgehalts aufweist.

3. Anlage zur Zucht von Insekten nach Anspruch 1 oder Anspruch 2, wobei das instrumentierte fahrerlose Transportfahrzeug (AGVI) als Sensor (2), der zur Messung eines für einen Zustand der Anlage charakteristischen Zustands geeignet ist, einen Bewegungssensor, eine optische und/oder Wärmekamera oder einen Bewegungswiderstandssensor aufweist.

4. Anlage zur Zucht von Insekten nach einem der vorangehenden Ansprüche, wobei der Sensor (2), der zur Messung eines charakteristischen Parameters eines Zustands der Anlage geeignet ist, gegenüber einem Körper (4) des instrumentierten fahrerlosen Transportfahrzeugs (AGVI) befestigt ist.

5. Anlage zur Zucht von Insekten nach einem der Ansprüche 1 bis 3, wobei der Sensor (3), der zur Messung eines charakteristischen Parameters eines Zustands der Anlage geeignet ist, gegenüber einem Körper (4) des instrumentierten fahrerlosen Transportfahrzeugs (AGVI) ausgerichtet sein oder verschoben werden kann.

6. Anlage zur Zucht von Insekten nach einem der vorangehenden Ansprüche, wobei das instrumentierte fahrerlose Transportfahrzeug (AGVI) eine drahtlose Übertragungsvorrichtung aufweist, die ausgelegt ist, um ein Signal zu senden, das der Messung des charakteristischen Parameters entspricht.

7. Anlage zur Zucht von Insekten nach einem der vorangehenden Ansprüche, wobei das instrumentierte fahrerlose Transportfahrzeug (AGVI) einen Informatikspeicher aufweist, in dem der charakteristische Parameter gespeichert ist und einen Kommunikationsport, der das drahtgebundene oder drahtlose Herunterladen aus dem Speicher erlaubt.

8. Anlage zur Zucht von Insekten nach einem der vorangehenden Ansprüche, die ferner feste Messsensoren des charakteristischen Parameters eines Zustands der Anlage aufweist.

9. Anlage zur Zucht von Insekten nach einem der vorangehenden Ansprüche, die ein zentrales Steuerungssystem des Zustands der Anlage (6) aufweist, das zum Empfang eines Signals, das für die Messung des charakteristischen Parameters repräsentativ ist, vom instrumentierten fahrerlosen Transportfahrzeug (AGVI) und zur Bestimmung, in Abhängigkeit von dem repräsentativen Signal, das mit einer Positionsinformation des instrumentierten fahrerlosen Transportfahrzeugs bei der Messung korreliert, des Vorliegens einer lokalen Anomalie im Zustand der Anlage geeignet ist.

10. Anlage zur Zucht von Insekten nach den Ansprüchen 8 und 9, wobei das zentrale Steuerungssystem des Zustands der Anlage (6) ferner zum Empfang von Signalen, die für Messungen des charakteristischen Parameters repräsentativ sind, von festen Sensoren und zur Bestimmung des Vorliegens einer lokalen Anomalie im Zustand der Anlage in Abhängigkeit von den repräsentativen Signalen geeignet ist, wobei jedes repräsentative Signal mit einer Identitätsinformation des festen Sensors, der es sendet, korreliert.

11. Anlage zur Zucht von Insekten nach Anspruch 9 oder Anspruch 10, die ein zentrales Führungssystem (1) aufweist, wobei das zentrale Führungssystem (1) zur Übertragung der Führungsinformationen an die fahrerlosen Transportfahrzeuge (AGV) und insbesondere an das instrumentierte fahrerlose Transportfahrzeug geeignet ist.

12. Anlage zur Zucht von Insekten nach den Ansprüchen 10 und 11, wobei das zentrale Führungssystem (1) ausgelegt ist, um an das instrumentierte fahrerlose Transportfahrzeug (AGVI) Führungsinformationen als Antwort auf eine lokale Anomalie zu übertragen, die auf der Grundlage eines Signals bestimmt wurde, das von einem der festen Sensoren gesendet wurde.

13. Anlage zur Zucht von Insekten 10 und 11, wobei das zentrale Führungssystem (1) ausgelegt ist, um an das instrumentierte fahrerlose Transportfahrzeug (AGVI) periodisch Führungsinformationen zu übertragen, damit es einen Teil der Anlage inspiziert, der nicht von einem festen Sensor der Anlage abgedeckt ist, um dort den charakteristischen Parameter zu messen.

14. Anlage zur Zucht von Insekten nach Anspruch 11 oder Anspruch 12, wobei das zentrale Führungssystem (1) ausgelegt ist, um an das instrumentierte fahrerlose Transportfahrzeug (AGVI) periodisch Führungsinformationen zu übertragen, damit es einen Teil der Anlage inspiziert, in dem die fahrerlosen Transportfahrzeuge während des Transports der Behälter (C1, C2) nicht verkehren.

15. Verfahren zur Steuerung eines Zustands einer Anlage zur Zucht von Insekten, die einen ersten Bereich (Z1) aufweist, in dem Insekten während ihres Wachstums in Behältern (C1, C2) gelagert sind, und einen zweiten Bereich (Z2), der mindestens einen Posten (P1, P2) aufweist, der für die Durchführung einer Maßnahme für die Insekten eines Behälters oder für den Behälter ausgelegt ist, wobei die Anlage fahrerlose Transportfahrzeuge (AGV) aufweist, die für die Verlagerung der Behälter in der Anlage ausgelegt sind, nämlich im ersten Bereich (Z1) und/oder im zweiten Bereich (Z2) und zwischen dem ersten Bereich (Z1) und dem zweiten Bereich (Z1),
wobei das Verfahren durch die Messung eines charakteristischen Parameters des Zustands der Anlage durch ein instrumentiertes fahrerloses Transportfahrzeug (AGVI) gekennzeichnet ist, das mit mindestens einem Sensor ausgestattet ist, der zur Durchführung der Messung des charakteristischen Parameters des Zustands der Anlage geeignet ist.

## Claims

1. Farm for rearing insects, said farm comprising a first zone (Z1) in which insects are stored during their growth in containers (C1, C2) and a second zone (Z2) comprising at least one station (P1,P2) configured for performing an operation on the insects of a container or on said container;
the farm comprising automated guided vehicles (AGV) configured for the movement of the containers in the farm, that is to say within the first zone (Z1), and/or within the second zone (Z2), and between the first zone (Z1) and the second zone (Z2), **characterized in that** said automated guided vehicles (AGV) comprise at least one instrument-equipped automated guided vehicle (IAGV), said instrument-equipped automated guided vehicle (IAGV) being equipped with at least one sensor (2) configured to perform a measurement of a parameter characteristic of a state of the farm.

2. Farm for rearing insects according to claim 1, wherein the instrument-equipped automated guided vehicle (IAGV) comprises, as sensor (2) configured for measuring a parameter characteristic of a state of the farm, a humidity sensor, a temperature sensor, or a sensor of the carbon dioxide level.

3. Farm for rearing insects according to claim 1 or claim 2, wherein the instrument-equipped automated guided (IAGV) vehicle comprises, as sensor (2) configured to measure a parameter characteristic of a state of the farm, a movement sensor, an optical or thermal camera, or a sensor of resistance to movement.

4. Farm for rearing insects according to one the preceding claims, wherein the sensor (2) configured for measuring a parameter characteristic of a state of the farm is fixed relative to a body (4) of the instrument-equipped automated guided vehicle (IAGV).

5. Farm for rearing insects according to one of claims 1 to 3, wherein the sensor (3) configured for measuring a parameter characteristic of a state of the farm may be oriented or moved relative to a body (4) of the instrument-equipped automated guided vehicle (IAGV).

6. Farm for rearing insects according to one the preceding claims, wherein the instrument-equipped automated guided vehicle (IAGV) comprises a device for wireless transmission configured to emit a signal corresponding to the measurement of the characteristic parameter.

7. Farm for rearing insects according to one the preceding claims, wherein the instrument-equipped automated guided vehicle (IAGV) comprises a computer memory in which the characteristic parameter is stored and a communication port enabling said memory to be unloaded by a wired or wireless channel.

8. Farm for rearing insects according to one the preceding claims, further comprising fixed sensors for measuring the parameter characteristic of a state of the farm.

9. Farm for rearing insects according to one the preceding claims, comprising a central system (6) for verifying the state of the farm configured to receive from the instrument-equipped automated guided vehicle (IAGV) a representation signal representing the measurement of the characteristic parameter and to determine, according to said representation signal correlated with an information item on position of the instrument-equipped automated guided vehicle at the time of said measurement, the presence of a local anomaly in the state of the farm.

10. Farm for rearing insects according to claims 8 and 9, wherein the central system (6) for verifying the state of the farm is furthermore adapted to receive from the fixed sensors representation signals representing measurements of the characteristic parameter and to determine the presence of a local anomaly in the state of the farm according to the representation signals, each representation signal being correlated with an item of information on identity of the fixed sensor which emits it.

11. Farm for rearing insects according to claim 9 or claim 10, comprising a central guidance system (1), said central guidance system (1) being configured to transmit guidance information to said automated guided vehicles (AGV) in particular for example to said instrument-equipped automated guided vehicle.

12. Farm for rearing insects according to claims 10 and 11 wherein said central guidance system (1) is configured to transmit to the instrument-equipped automated guided vehicle (IAGV) guidance information in response to a local anomaly determined on the basis of a signal emitted by one of the fixed sensors.

13. Farm for rearing insects according to claim 10 and 11, wherein said central guidance system (1) is configured to perform periodic transmission to the instrument-equipped automated guided vehicle (IAGV) of guidance information in order to make it inspect part of the farm which is not covered by a fixed sensor of the farm to measure the characteristic parameter there.

14. Farm for rearing insects according to claim 11 or claim 12, wherein said central guidance system (1) is configured to perform periodic transmission to the instrument-equipped automated guided vehicle (IAGV), of guidance information in order to make it inspect part of the farm in which the automated guided vehicles do not pass when transporting containers (C1,C2).

15. Method for verifying a state of a farm for rearing insects comprising a first zone (Z1) in which the insects are stored during their growth in containers (C1, C2) and a second zone (Z2) comprising at least one station (P1,P2) configured for the carrying out of an operation on the insects of a container or on said container, the farm comprising automated guided vehicles (AGV) configured for the movement of the containers in the farm, that is to say within the first zone (Z1), and/or within the second zone (Z2), and between the first zone (Z1) and the second zone (Z2),
said method being **characterized by** the measurement of a parameter characteristic of the state of the farm by an instrument-equipped automated guided vehicle (IAGV) equipped with at least one sensor configured to make the measurement of said parameter characteristic of the state of the farm.
